# EUROPEAN PATENT APPLICATION

(11) **EP 4 574 811 A1**
(43) Date of publication of application: **25.06.2025**
(21) Application number: 23219783.0
(22) Date of filing: 22.12.2023
(51) Int. Cl.: C07C 217/84, A61P 35/00

(54) **CRYSTALLINE FORMS OF ELACESTRANT DIHYDROCHLORIDE**

(71) Applicant: SANDOZ AG, 4051 Basel (CH)
(72) Inventor: The designation of the inventor has not yet been filed
(74) Representative: Stefánsson, Stefán Einar

(57) **Abstract**

The present invention relates to novel crystalline forms of Elacestrant dihydrochloride. The present disclosure also generally relates to a pharmaceutical composition comprising the crystalline forms of Elacestrant dihydrochloride of the present invention, as well of methods of using the same in the treatment of conditions and diseases driven by estrogen, preferably for the treatment of breast cancer.

## Description

### FIELD OF INDUSTRIAL APPLICABILITY

The present disclosure encompasses novel solid state forms of Elacestrant dihydrochloride, processes for preparation, pharmaceutical compositions and medical use thereof.

### BACKGROUND OF THE DISCLOSURE

Elacestrant dihydrochloride, (6R)-6-(2-(ethyl((4-(2-(ethylamino)ethyl)phenyl)-methylamino)-4-methoxyphenyl)-5,6,7,8-Tetrahydronaphthalen-2-ol dihydrochloride, has the chemical structure disclosed below:

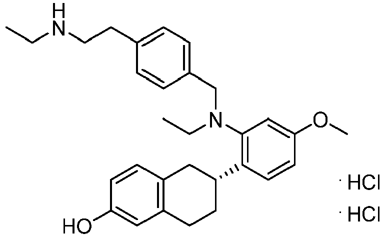

Elacestrant has been shown to be a selective estrogen receptor modulator particularly useful for the treatment of metastatic breast cancer. Tablets comprising Elacestrant dihydrochloride have recently been approved for the treatment of postmenopausal women or adult men, with ER-positive, HER2-negative, ESR1-mutated advanced or metastatic breast cancer and are marketed under the brand name ORSERDU^{™}.

The compound in its free base form is described in International Publication No. WO 2004/058682. Elacestrant dihydrochloride has been described in International Publication No. WO 2016/176665 and specific crystalline forms of the dihydrochloride salt of Elacestrant are disclosed in International Publication Nos. WO 2018/0129419 and WO 2020/010216. Furthermore, co-crystals of Elacestrant dihydrochloride with urea have been disclosed in International Publication Nos. WO 2023/064519.

Polymorphism, the occurrence of different crystalline forms, is a property of some molecules, salts and molecular complexes. A single molecule, salt or molecular complex may give rise to a variety of polymorphs having distinct crystal structures and physical properties like melting point, thermal behaviors (e.g., measured by thermogravimetric analysis ("TGA"), or differential scanning calorimetry ("DSC"), X-ray diffraction (XRD) pattern, infrared absorption fingerprint, and solid state (13C) NMR spectrum. One or more of these techniques may be used to distinguish different polymorphic forms of a compound.

Different solid state forms (including solvated forms) of an active pharmaceutical ingredient may possess different properties. Such variations in the properties of different salts and solid state forms and solvates may provide a basis for improving formulation, for example, by facilitating better processing or handling characteristics, changing the dissolution profile in a favorable direction, or improving stability (polymorph as well as chemical stability) and shelf-life. These variations in the properties of different salts and solid state forms may also offer improvements to the final dosage form, for instance, if they serve to improve bioavailability. Different salts and solid state forms and solvates of an active pharmaceutical ingredient may also give rise to a variety of polymorphs or crystalline forms, which may in turn provide additional opportunities to assess variations in the properties and characteristics of a solid active pharmaceutical ingredient.

Discovering new solid state forms and solvates of a pharmaceutical product may yield materials having desirable processing properties, such as ease of handling, ease of processing, storage stability, and ease of purification or as desirable intermediate crystal forms that facilitate conversion to other polymorphic forms. New solid state forms of a pharmaceutically useful compound can also provide an opportunity to improve the performance characteristics of a pharmaceutical product. It enlarges the repertoire of materials that a formulation scientist has available for formulation optimization, for example by providing a product with different properties, including a different crystal habit, higher crystallinity, or polymorphic stability, which may offer better processing or handling characteristics, improved dissolution profile, or improved shelf-life (chemical/physical stability). For at least these reasons, there is a need for additional solid state forms (including solvated forms) of Elacestrant dihydrochloride.

### SUMMARY OF THE DISCLOSURE

The present disclosure provides two novel crystalline forms of Elacestrant dihydrochloride, form A and form B, processes for preparing both forms, and pharmaceutical compositions comprising crystalline Elacestrant dihydrochloride form A, B.

The present disclosure also relates to the use of crystalline Elacestrant dihydrochloride disclosed herein for the preparation of a medicament, preferably for the treatment of Gaucher disease.

The present invention further provides a pharmaceutical composition comprising crystalline Elacestrant hydrochloride of the present disclosure and at least one pharmaceutically acceptable excipient.

The present invention also provides a method of treating Gaucher disease, comprising administering a therapeutically effective amount of crystalline Elacestrant hydrochloride of the present disclosure, or at least one of the above pharmaceutical compositions to a person suffering from Gaucher disease.

### Definitions

In the context of the present invention the following abbreviations have the indicated meaning, unless explicitly stated otherwise:
- PXRD: powder X-ray diffraction/ diffractogram
- TGA: thermogravimetric analyses
- DSC: differential scanning calorimetry
- DMA: dimethylacetamide
- RT: room temperature
- RH: relative humidity
- m: mass
- Δm: mass change
- hr: heating rate

As used herein the term "room temperature" indicates that the applied temperature is not critical and that no exact temperature value has to be kept. Usually, "room temperature" is understood to mean temperatures between 15 and 25 °C [see e.g. European Pharmacopoeia 8.3, 1.2 (2015)].

As used herein, the term "mother liquor" refers to the solution remaining after crystallization of a solid.

The term "non-hygroscopic" as used herein refers to compounds showing a mass change of less than 2% (w/w) based on the weight of the compound in the range of from 0 to 95% relative humidity at (25.0 ± 0.1) °C.

The term "needle" as used herein with regards to crystal shape refers to acicular, thin and highly elongated crystals having similar length and width.

The term "essentially the same" with reference to PXRD means that variabilities in peak positions and relative intensities of the peaks are to be taken into account. For example, a typical precision of the 2-Theta values is in the range of ± 0.1° 2-Theta. Thus, a diffraction peak that usually appears at 6.0° 2-Theta for example can appear between 5.9° and 6.1° 2-Theta on most X-ray diffractometers under standard conditions. Furthermore, one skilled in the art will appreciate that relative peak intensities will show inter-apparatus variability as well as variability due to degree of crystallinity, preferred orientation, sample preparation and other factors known to those skilled in the art and should be taken as qualitative measure only.

As used herein, the term "substantially pure" with reference to a particular polymorphic form means that the polymorphic form includes less than 10%, preferably less than 5%, more preferably less than 3%, most preferably less than 1% by weight of any other physical forms of the compound.

### BRIEF DESCRIPTION OF THE DRAWINGS

Figure 1. illustrates a characteristic X-ray powder diffraction pattern (XRPD) of Elacestrant dihydrochloride form A.
Figure 2: illustrates a representative DSC curve of Elacestrant dihydrochloride A. The x-axis shows the temperature in degree Celsius (°C), the y-axis shows the heat flow rate in Watt per gram (W/g) with endothermic peaks going up.
Figure 3. illustrates the thermogravimetric differential thermal analysis (TG-DTA) thermogram of crystalline Elacestrant dihydrochloride form A.
Figure 4. illustrates a representative GMS isotherm of Elacestrant dihydrochloride form A in the range of from 1 to 90% relative humidity. The x-axis displays the relative humidity in percent (%) measured at a temperature of (25.0 ± 0.1)°C, the y-axis displays the equilibrium mass change in weight percent (w-%).
Figure 5. illustrates a characteristic X-ray powder diffraction pattern (XRPD) of Elacestrant dihydrochloride form B.
Figure 6: illustrates a representative DSC curve of Elacestrant dihydrochloride form B. The x-axis shows the temperature in degree Celsius (°C), the y-axis shows the heat flow rate in Watt per gram (W/g) with endothermic peaks going up.
Figure 7. illustrates the thermogravimetric differential thermal analysis (TG-DTA) thermogram of crystalline Elacestrant dihydrochloride form B.
Figure 8. illustrates a representative GMS isotherm of Elacestrant dihydrochloride form B in the range of from 1 to 90% relative humidity. The x-axis displays the relative humidity in percent (%) measured at a temperature of (25.0 ± 0.1)°C, the y-axis displays the equilibrium mass change in weight percent (w-%).

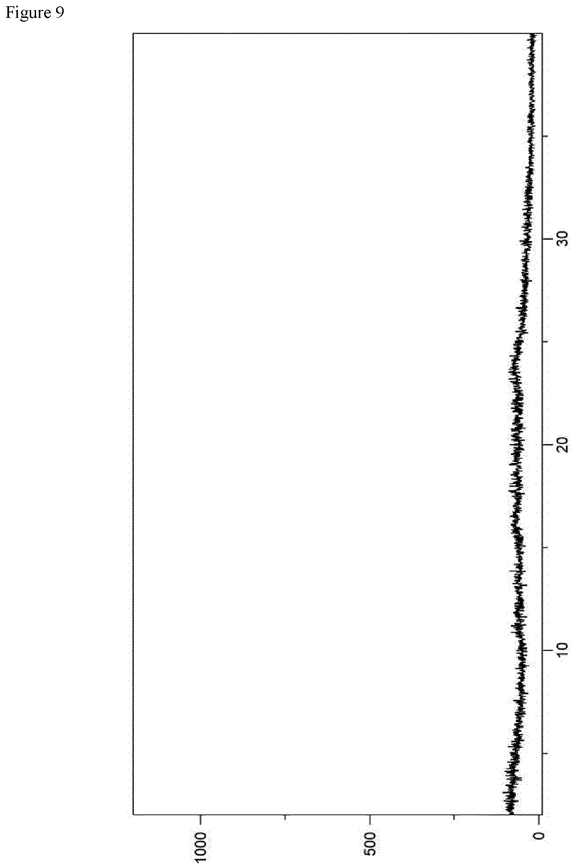

Figure 9. illustrates a characteristic X-ray powder diffraction pattern (XRPD) of amorphous Elacestrant dihydrochloride.

### DETAILED DESCRIPTION OF THE DISCLOSURE

The present disclosure comprises novel crystalline forms of Elacestrant dihydrochloride, processes for preparation thereof, pharmaceutical compositions and use thereof.

A solid state form (or polymorph) may be referred to herein as polymorphically pure or as substantially free of any other solid state (or polymorphic) forms. As used herein in this context, the expression "substantially free of any other forms" will be understood to mean that the solid state form contains about 20% (w/w) or less, about 10% (w/w) or less, about 5% (w/w) or less, about 2% (w/w) or less, about 1% (w/w) or less, or about 0% of any other forms of the subject compound as measured, for example, by PXRD. Thus, a crystalline polymorph of Elacestrant dihydrochloride described herein as substantially free of any other solid state forms would be understood to contain greater than about 80% (w/w), greater than about 90% (w/w), greater than about 95% (w/w), greater than about 98% (w/w), greater than about 99% (w/w), or about 100% of the subject crystalline polymorph of Elacestrant dihydrochloride. In some embodiments of the disclosure, the described crystalline polymorph of Elacestrant dihydrochloride may contain from about 1% to about 20% (w/w), from about 5% to about 20% (w/w), or from about 5% to about 10% (w/w) of one or more other crystalline polymorph of the same Elacestrant dihydrochloride.

Depending on which other crystalline polymorphs a comparison is made, the crystalline polymorphs of Elacestrant dihydrochloride of the present disclosure may have advantageous properties selected from at least one of the following: ease of preparation, chemical purity, flowability, solubility, dissolution rate, morphology or crystal habit, stability, such as chemical stability as well as thermal and mechanical stability with respect to polymorphic conversion, stability towards dehydration and/or storage stability, low content of residual solvent, a lower degree of hygroscopicity, flowability, and advantageous processing and handling characteristics such as compressibility and bulk density.

A solid state form, such as a crystal form or an amorphous form, may be referred to herein as being characterized by graphical data "as depicted in" or "as substantially depicted in" a Figure. Such data include, for example, powder X-ray diffractograms and solid state NMR spectra. As is well-known in the art, the graphical data potentially provides additional technical information to further define the respective solid state form (a so-called "fingerprint") which cannot necessarily be described by reference to numerical values or peak positions alone. In any event, the skilled person will understand that such graphical representations of data may be subject to variations, e.g., in peak relative intensities and peak positions due to certain factors such as, but not limited to, variations in instrument response and variations in sample concentration and purity, which are well known to the skilled person. Nonetheless, the skilled person would readily be capable of comparing the graphical data in the Figures herein with graphical data generated for an unknown crystal form and confirm whether the two sets of graphical data are characterizing the same crystal form or two different crystal forms. A crystal form of Elacestrant dihydrochloride referred to herein as being characterized by graphical data "as depicted in" or "as substantially depicted in" a Figure will thus be understood to include any crystal forms of Elacestrant dihydrochloride characterized with the graphical data having such small variations, as are well known to the skilled person, in comparison with the Figure.

As used herein, and unless stated otherwise, the term "anhydrous" in relation to crystalline forms of Elacestrant dihydrochloride, relates to a crystalline form of Elacestrant dihydrochloride which does not include any crystalline water (or other solvents) in a defined, stoichiometric amount within the crystal. Moreover, an "anhydrous" form would generally not contain more than 1% (w/w), of either water or organic solvents as measured for example by TGA.

The term "solvate," as used herein and unless indicated otherwise, refers to a crystal form that incorporates a solvent in the crystal structure. When the solvent is water, the solvate is often referred to as a "hydrate." The solvent in a solvate may be present in either a stoichiometric or in a non-stoichiometric amount.

As used herein, unless stated otherwise, the PXRD measurements are taken using Cu-Kalpha1,2 radiation (wavelength 1.5419 Å).

As used herein, unless stated otherwise, DSC analysis is carried out at a heating rate of 10 °C/min from 25 °C to 200 °C, preferably with a nitrogen flow of 50 ml/minute (sample purge).

As used herein, unless stated otherwise, TGA analysis is carried out at a heating rate of 10 °C/min from 25 °C to 200 °C, preferably with a nitrogen flow of 30 ml/minute (sample purge).

An object, e.g., a reaction mixture, may be characterized herein as being at, or allowed to come to "room temperature" or "ambient temperature", often abbreviated as "RT." This means that the temperature of the object is close to, or the same as, that of the space, e.g., the room or fume hood, in which the thing is located. Typically, room temperature is from about 20°C to about 30°C, or about 22°C to about 27°C, or about 25°C.

The amount of solvent employed in a chemical process, e.g., a reaction or crystallization, may be referred to herein as a number of "volumes" or "vol" or "V." For example, a material may be referred to as being suspended in 10 volumes (or 10 vol or 10V) of a solvent. In this context, this expression would be understood to mean milliliters of the solvent per gram of the material being suspended, such that suspending a 5 grams of a material in 10 volumes of a solvent means that the solvent is used in an amount of 10 milliliters of the solvent per gram of the material that is being suspended or, in this example, 50 mL of the solvent. In another context, the term "v/v" may be used to indicate the number of volumes of a solvent that are added to a liquid mixture based on the volume of that mixture. For example, adding solvent X (1.5 v/v) to a 100 ml reaction mixture would indicate that 150 mL of solvent X was added.

A process or step may be referred to herein as being carried out "overnight." This refers to a time interval, e.g., for the process or step, that spans the time during the night, when that process or step may not be actively observed. This time interval is from about 8 to about 20 hours, or about 10-18 hours, in some cases about 16 hours.

As used herein, the term "mother liquor" refers to the solution remaining after crystallization of a solid from said solution.

As used herein, the term "reduced pressure" refers to a pressure that is less than atmospheric pressure. For example, reduced pressure is about 10 mbar to about 50 mbar.

As used herein and unless indicated otherwise, the term "ambient conditions" refer to atmospheric pressure and a temperature of 22-24°C.

Various analytical methods may be used for characterization.

### Powder X-ray diffraction

Powder X-ray diffraction was performed with a PANalytical X'Pert PRO diffractometer equipped with a theta/theta coupled goniometer in transmission geometry, Cu-Kalpha1,2 radiation (wavelength 0.15419 nm) with a focusing mirror and a solid state PIXcel detector. Diffractograms were recorded at a tube voltage of 45 kV and a tube current of 40 mA, applying a step size of 0.013° 2-theta with 40s per step (255 channels) in the angular range of 2° to 40° 2-Theta at ambient conditions. A typical precision of the 2-Theta values is in the range of ± 0.2° 2-Theta, preferably of ± 0.1° 2-Theta.

### Differential Scanning Calorimetry (DSC)

DSC was performed on a Mettler Polymer DSC R instrument. The sample was heated in a 40 microliter aluminum pan with a pierced aluminum lid from 25 to 200 °C at a rate of 10 K/min. Nitrogen (purge rate 50 mL/min) was used as purge gas.

### Thermogravimetric Analysis (TGA)

TGA was performed on a Mettler TGA/DSC 1 instrument. The sample was heated in a 100 microliter aluminum pan closed with an aluminum lid. The lid was automatically pierced at the beginning of the measurement. The sample was heated from 25 to 200 °C at a rate of 10 K/min. Nitrogen (purge rate 30 mL/min) was used as purge gas.

### Dynamic Vapor Sorption (DVS):

Moisture sorption isotherms were recorded with an SPSx-1µ moisture sorption analyzer (ProUmid, Ulm). Measurement cycles were started at ambient relative humidity. Relative humidity was then decreased to 5% RH in 5% steps, followed by a further decrease to 3% RH and to approximately 1% RH. Afterwards RH was increased from 1% to 90% RH in a sorption cycle and decreased to 1 % in a desorption cycle in 5% steps. Finally, RH was increased ambient relative humidity again. The time per step was set to a minimum of 2 hours and a maximum of 6 hours. If an equilibrium condition with a constant mass of ± 0.01% within 1 hour was reached before the maximum time for all examined samples the sequential humidity step was applied before the maximum time of 6 hours. If no equilibrium was achieved the consecutive humidity step was applied after the maximum time of 6 hours. The temperature was 25 ± 0.1 °C.

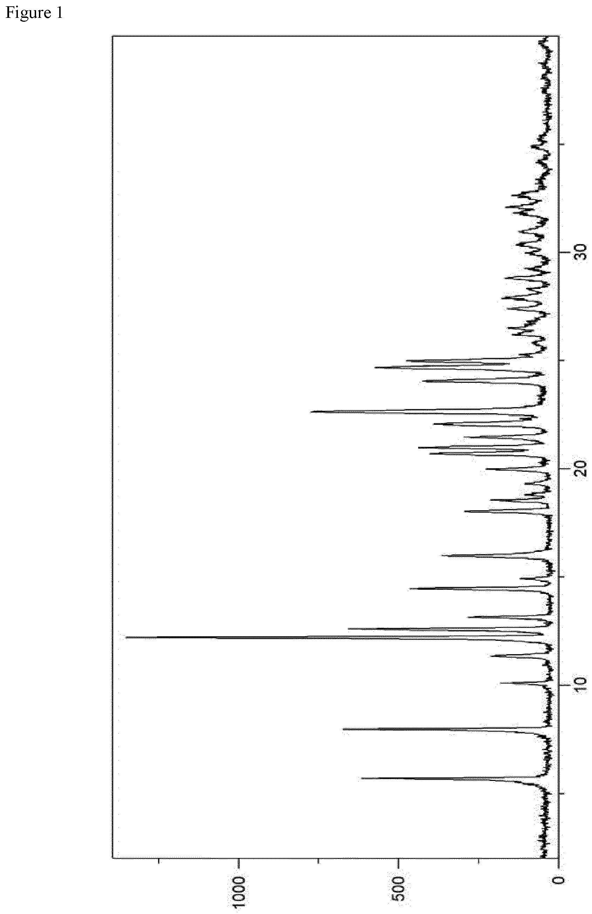

The present disclosure includes a crystalline Elacestrant dihydrochloride form A, designated form A. Crystalline form A may be characterized by data selected from one or more of the following: an X-ray powder diffraction pattern substantially as depicted in Figure 1; an PXRD pattern having peaks at 5.7, 8.0, 12.2, 12.6, and 22.6 degrees 2-theta ± 0.2 degrees 2-theta; and combinations of these data.

Crystalline form A may be further characterized by an X-ray powder diffraction pattern having peaks at 5.7, 8.0, 12.2, 12.6, and 22.6 degrees 2-theta ± 0.2 degrees 2-theta, and also having any one, two, three, four or five additional peaks selected from 14.5, 16.0, 21.0, 24.1, and 24.7 degrees 2-theta ± 0.2 degrees 2-theta.

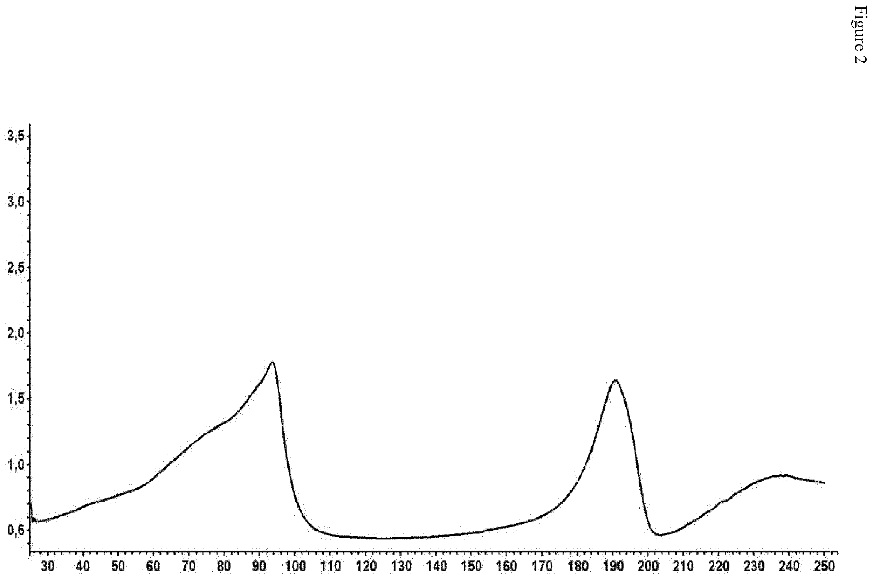

Alternatively, or additionally, form A is characterized by having a DSC curve substantially as depicted in Figure 2; comprising an endothermic peak with an onset temperature of about 68 °C and 177 °C and a peak maximum temperature of about 93.5 °C and 190 °C.

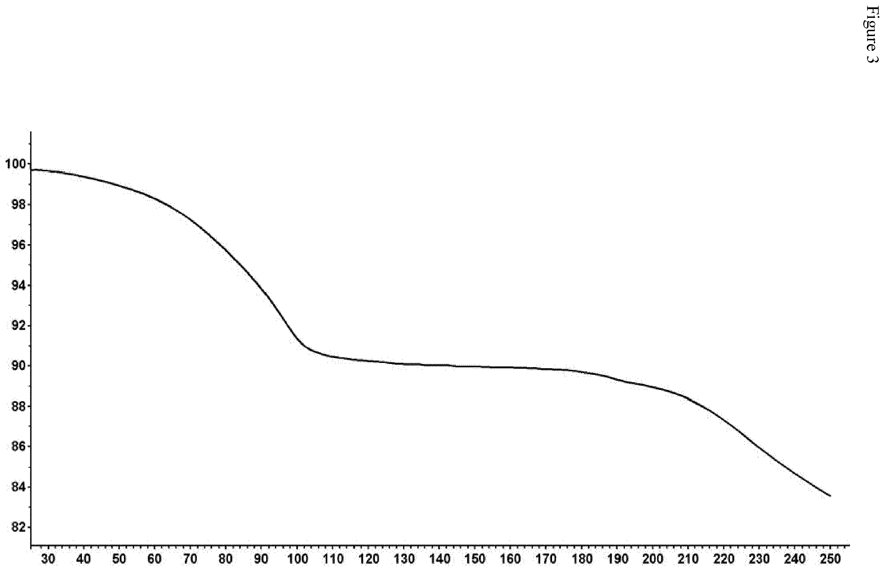

Alternatively, or additionally, form A is characterized by having a TGA curve substantially as depicted in Figure 3. The TGA curve shows a mass loss of 9,4% until 110°C.

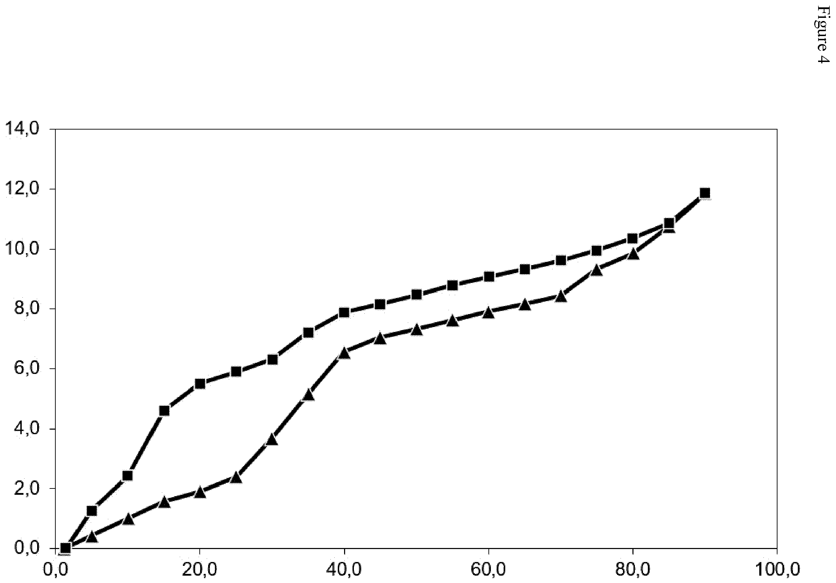

Alternatively, or additionally, form A is characterized by having a DVS curve substantially as depicted in Figure 4.

Crystalline form A may be a hydrate. In embodiments, crystalline form A may contain about 9.1 % to about 9.7 % water, or about 9.4 % water by weight.

In embodiments, crystalline form A may be polymorphically pure.

Surprisingly it was found that pure form A dissolves in water at a concentration of about 34.5 mg/ml at 24 °C providing a clear solution.

In a further aspect the present invention relates to a process for the preparation of crystalline form A. Surprisingly it has been found that amorphous Elacestrant dihydrochloride can be transformed into crystalline form B acid under mild neat conditions.

In a preferred aspect the present invention relates to a process for the preparation of crystalline form A comprising:
(i) dispersing amorphous Elacestrant free base in a solvent
(ii) adding hydrochloride acid and stirring until crystallization has completed
(iii) isolating the crystals obtained in (ii)
(iv) drying the isolated crystals obtained in (iii)
(v) optionally milling the crystals obtained in (iv)
(vi) optionally sieving the crystals of obtained in (iv, v).

The process wherein the solvent is selected from alcohols, preferentially the solvent is selected from C2-C6 aliphatic alcohols, most preferentially the solvent is 2-propanol or 1-butanol.

The process wherein aqueous hydrochloric acid added, preferentially about 37% aqueous hydrochloric is added.

The process wherein the solution is stirred for a period within a range from about 12 hours to about 24 hours, preferentially within a range from about 16 hours to about 20 hours, most preferentially about 18 hours.

The process wherein the form A crystals obtained in (ii) are isolated by conventional methods such as filtering or centrifugation.

The process wherein isolated form A crystals obtained in (iii) are dried for a period within a range from about 12 hours to about 24 hours, preferentially within a range from about 16 hours to about 20 hours, most preferentially about 18 hours.

The process wherein isolated form A crystals obtained in (iii) are dried at a temperature within a range from about 10 °C to about 30 °C, preferentially within a range from about 15 °C to about 25 °C, most preferentially at RT.

The process wherein the particle size distribution of the form A crystals obtained in (iv) is optionally controlled by a milling and/or sieving process.

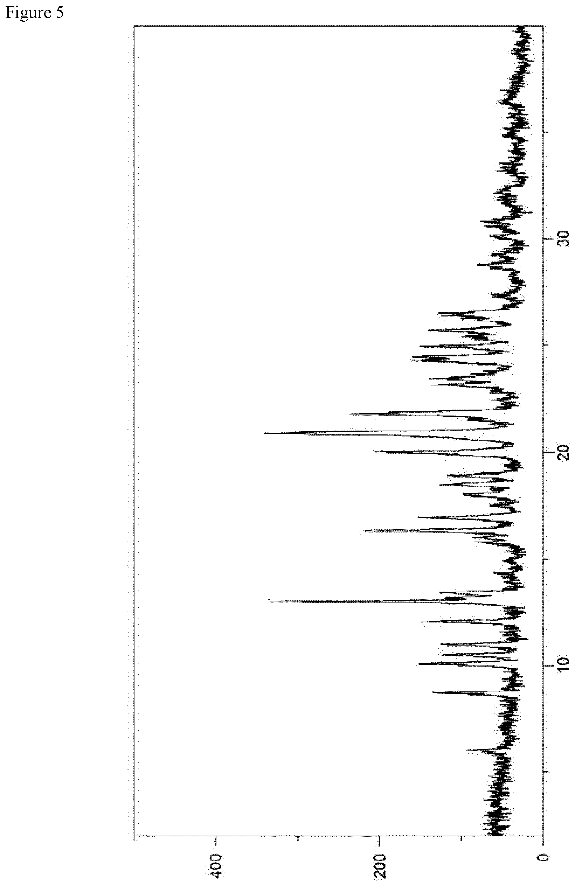

The present disclosure includes a crystalline Elacestrant dihydrochloride form B, designated form B. Crystalline form B may be characterized by data selected from one or more of the following: an X-ray powder diffraction pattern substantially as depicted in Figure 5; an PXRD pattern having peaks at 8.7, 10.1, 13.0, 16.3, and 21.8 degrees 2-theta ± 0.2 degrees 2-theta; and combinations of these data. Crystalline form B may be further characterized by an X-ray powder diffraction pattern having peaks at 8.7, 10.1, 13.0, 16.3, and 21.8 degrees 2-theta ± 0.2 degrees 2-theta, and also having any one, two, three, four or five additional peaks selected from 10.5, 12.1, 20.0, 24.5, and 25.7 degrees 2-theta ± 0.2 degrees 2-theta.

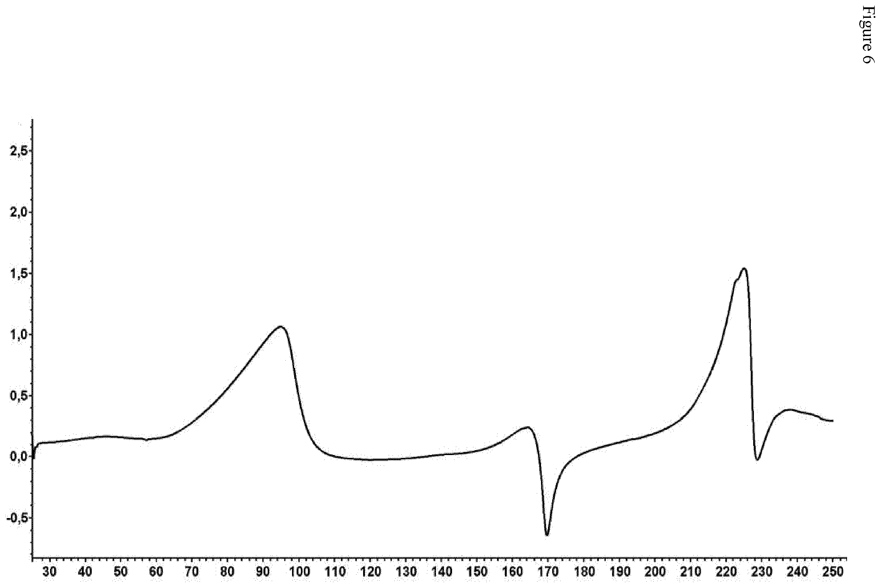

Alternatively, or additionally, form B is characterized by having a DSC curve substantially as depicted in Figure 6; comprising an endothermic peak with an onset temperature of about 68 °C, 152 °C and 213 °C and a peak maximum temperature of about 95 °C, 164 °C and 225 °C. Furthermore, it shows an exothermic peak with an onset of about 167 °C and a peak minimum temperature of about 170 °C.

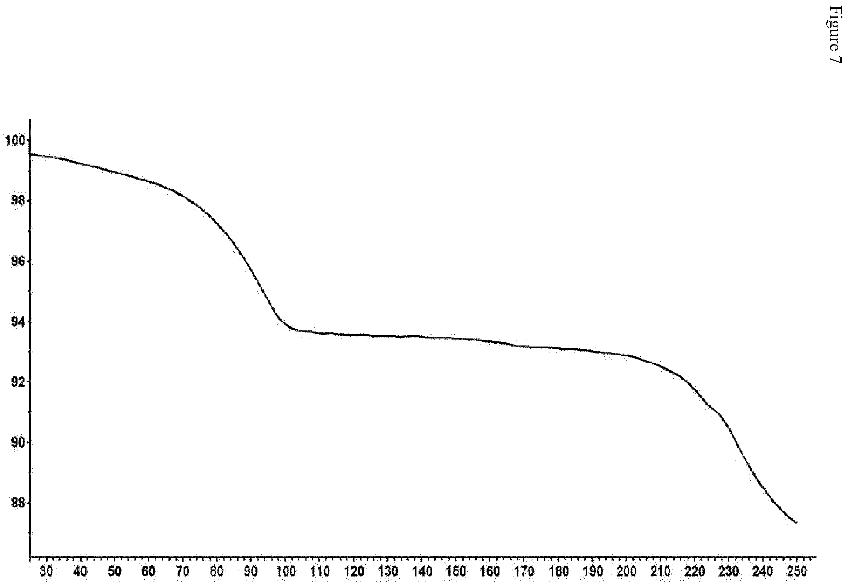

Alternatively, or additionally, form B is characterized by having a TGA curve substantially as depicted in Figure 7. The TGA curve shows a mass loss of 5,8% until 110°C.

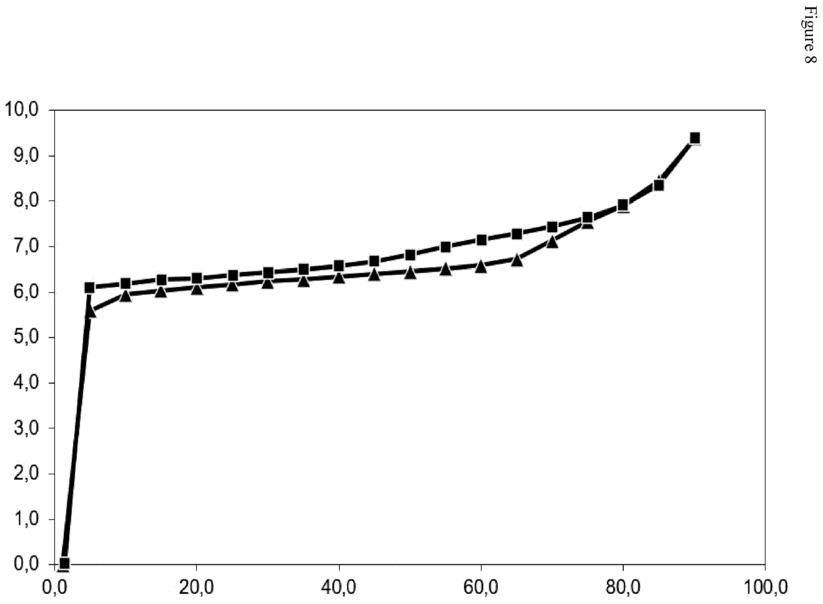

Alternatively, or additionally, form B is characterized by having a DVS curve substantially as depicted in Figure 8.

Crystalline form B may be a hydrate, preferably a dihydrate. In embodiments, crystalline form B may contain about 5.5 % to about 6.1 % water, or about 5.8 % water by weight.

In embodiments, crystalline form B may be polymorphically pure.

Surprisingly it was found that pure form B dissolves in water at a concentration of about 34.5 mg/ml at 24 °C providing a clear solution. Furthermore, it was found that pure form B dissolves in water even at a concentration of about 51.9 mg/ml at 24 °C providing a clear solution, however, from the resulting supersaturated solution subsequently precipitation of Elacestrant dihydrochloride was occurred.

In a further aspect the present invention relates to a process for the preparation of crystalline form B. Surprisingly it has been found that amorphous Elacestrant dihydrochloride can be transformed into crystalline form B acid under mild neat conditions.

Surprisingly it was found that form B can be obtained starting from amorphous Elacestrant dihydrochloride by controlled storage under humid conditions in absence of any solvent.

Therefore, in In a preferred aspect the present invention relates to a process for the preparation of crystalline form B comprising:
(i) storing amorphous Elacestrant dihydrochloride at a humidity higher than 60% and at a temperature below 40 °C
(ii) optionally checking crystallinity
(iii) optionally milling the crystals obtained in (i);
(iv) optionally sieving the crystals obtained in (i, ii).

The process wherein the amorphous Elacestrant dihydrochloride is stored at a humidity in a range of from about 70% to 85%, preferable from about 70% to 85%, at a temperature in a range of from about 15 °C to 35 °C, preferably, in a range of from about 20 °C to 30 °C. Most preferable the amorphous Elacestrant hydrochloride is stored at a humidity in a range of from about 75% to 80% at RT.

The process wherein the amorphous Elacestrant dihydrochloride is stored for a period in a range of from about 3 to 10 days, preferable from about 5 to 8 days, most preferable from about 6 to 7 days.

The process wherein the crystallinity is optionally checked by PXRD.

The process wherein the particle size distribution of the form B crystals obtained in (i) is optionally controlled by a milling and/or sieving process.

In one embodiment of the disclosure, crystalline form B is provided substantially polymorphically and/or chemically pure.

The crystalline form B in substantially pure form may be employed in pharmaceutical compositions which may optionally include one or more other components selected, for example, from the group consisting of excipients, carriers, and one of other active pharmaceutical ingredients active chemical entities of different molecular structure.

In another embodiment of the disclosure, form B is provided as component of a mixture together with other solid state forms of Elacestrant dihydrochloride. Such a mixture of solid state crystalline forms of Elacestrant dihydrochloride comprising form B may be employed in pharmaceutical compositions which may optionally include one or more other components selected, for example, from the group consisting of excipients, carriers, and one of other active pharmaceutical ingredients active chemical entities of different molecular structure.

In yet another embodiment of the disclosure, form B is provided as component of a mixture together with amorphous Elacestrant dihydrochloride. A mixture amorphous Elacestrant dihydrochloride comprising form B may be employed in pharmaceutical compositions which may optionally include one or more other components selected, for example, from the group consisting of excipients, carriers, and one of other active pharmaceutical ingredients active chemical entities of different molecular structure. In another aspect, the invention relates to a pharmaceutical composition comprising a therapeutically effective amount of form B and at least one pharmaceutically acceptable excipient.

In further aspect, the invention relates to a pharmaceutical composition comprising form B and at least one pharmaceutically acceptable excipient.

In another preferred aspect, the pharmaceutical composition is suitable for oral administration, most preferable a tablet.

In further aspect, the present disclosure also encompasses the use of form B for the manufacture of a medicament, preferably for the treatment of breast cancer.

The present disclosure provides the above described crystalline polymorphs of Elacestrant dihydrochloride for use in the preparation of pharmaceutical compositions comprising Elacestrant dihydrochloride.

The present disclosure also encompasses the use of crystalline polymorphs of Elacestrant dihydrochloride of the present disclosure for the preparation of pharmaceutical compositions of crystalline polymorph Elacestrant dihydrochloride.

The present disclosure includes processes for preparing the above mentioned pharmaceutical compositions. The processes include combining any one or a combination of the crystalline polymorphs of Elacestrant dihydrochloride of the present disclosure with at least one pharmaceutically acceptable excipient.

The present disclosure further includes a crystalline polymorph of Elacestrant dihydrochloride of the present disclosure for use as a medicament, preferably wherein the medicament is for the treatment of breast cancer.

The present disclosure further provides a method of treating of breast cancer comprising administering a therapeutically effective amount of a crystalline polymorph of Elacestrant dihydrochloride of the present disclosure to a subject in need of treatment.

Diluents increase the bulk of a solid pharmaceutical composition, and can make a pharmaceutical dosage form containing the composition easier for the patient and caregiver to handle. Diluents for solid compositions include, for example, microcrystalline cellulose (e.g. Avicel(R)), microfine cellulose, lactose, starch, pregelatinized starch, calcium carbonate, calcium sulfate, sugar, dextranes, dextrin, dextrose, dibasic calcium phosphate dihydrate, tribasic calcium phosphate, kaolin, magnesium carbonate, magnesium oxide, maltodextrin, mannitol, polymethacrylates (e.g. Eudragit(R)), potassium chloride, powdered cellulose, sodium chloride, sorbitol, and talc.

Solid pharmaceutical compositions that are compacted into a dosage form, such as a tablet, can include excipients whose functions include helping to bind the active ingredient and other excipients together after compression. Binders for solid pharmaceutical compositions include acacia, alginic acid, carbomer (e.g. carbopol), carboxymethylcellulose sodium, dextrin, ethyl cellulose, gelatin, guar gum, hydrogenated vegetable oil, hydroxyethyl cellulose, hydroxypropyl cellulose (e.g. Klucel(R)), hydroxypropyl methyl cellulose (e.g. Methocel(R)), liquid glucose, magnesium aluminum silicate, maltodextrin, methylcellulose, polymethacrylates, povidone (e.g. Kollidon(R), Plasdone(R)), pregelatinized starch, sodium alginate, and starch.

The dissolution rate of a compacted solid pharmaceutical composition in the patient's stomach can be increased by the addition of a disintegrant to the composition. Disintegrants include alginic acid, carboxymethylcellulose calcium, carboxymethylcellulose sodium (e.g. Ac- Di-Sol(R), Primellose(R)), colloidal silicon dioxide, croscarmellose sodium, crospovidone (e.g. Kollidon(R), Polyplasdone(R)), guar gum, magnesium aluminum silicate, methyl cellulose, microcrystalline cellulose, polacrilin potassium, powdered cellulose, pregelatinized starch, sodium alginate, sodium starch glycolate (e.g. Explotab(R)), and starch.

Glidants can be added to improve the flowability of a non-compacted solid composition and to improve the accuracy of dosing. Excipients that can function as glidants include colloidal silicon dioxide, magnesium trisilicate, powdered cellulose, starch, talc, and tribasic calcium phosphate.

When a dosage form such as a tablet is made by the compaction of a powdered composition, the composition is subjected to pressure from a punch and dye. Some excipients and active ingredients have a tendency to adhere to the surfaces of the punch and dye, which can cause the product to have pitting and other surface irregularities. A lubricant can be added to the composition to reduce adhesion and ease the release of the product from the dye. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc, and zinc stearate.

Flavoring agents and flavor enhancers make the dosage form more palatable to the patient. Common flavoring agents and flavor enhancers for pharmaceutical products that can be included in the composition of the present disclosure include maltol, vanillin, ethyl vanillin, menthol, citric acid, fumaric acid, ethyl maltol, and tartaric acid.

The solid compositions of the present disclosure include powders, granulates, aggregates, and compacted compositions. The dosages include dosages suitable for oral, buccal, rectal, parenteral (including subcutaneous, intramuscular, and intravenous), inhalant, and ophthalmic administration. Although the most suitable administration in any given case will depend on the nature and severity of the condition being treated, in embodiments the route of administration is oral. The dosages can be conveniently presented in unit dosage form and prepared by any of the methods well-known in the pharmaceutical arts.

Dosage forms include solid dosage forms like tablets, powders, capsules, suppositories, sachets, troches, and lozenges, as well as liquid syrups, suspensions, and elixirs.

The dosage form of the present disclosure can be a capsule containing the composition, such as a powdered or granulated solid composition of the disclosure, within either a hard or soft shell. The shell can be made from gelatin and optionally contain a plasticizer such as glycerin and/or sorbitol, an opacifying agent and/or colorant.

The active ingredient and excipients can be formulated into compositions and dosage forms according to methods known in the art.

A composition for tableting or capsule filling can be prepared by wet granulation. In wet granulation, some or all of the active ingredients and excipients in powder form are blended and then further mixed in the presence of a liquid, typically water, that causes the powders to clump into granules. The granulate is screened and/or milled, dried, and then screened and/or milled to the desired particle size. The granulate can then be tableted, or other excipients can be added prior to tableting, such as a glidant and/or a lubricant.

A tableting composition can be prepared conventionally by dry blending. For example, the blended composition of the actives and excipients can be compacted into a slug or a sheet and then comminuted into compacted granules. The compacted granules can subsequently be compressed into a tablet.

Alternatively, a blended composition can be compressed directly into a compacted dosage form using direct compression techniques. Direct compression produces a more uniform tablet without granules. Excipients that are particularly well suited for direct compression tableting include microcrystalline cellulose, spray dried lactose, dicalcium phosphate dihydrate, and colloidal silica. The proper use of these and other excipients in direct compression tableting is known to those in the art with experience and skill in particular formulation challenges of direct compression tableting.

A capsule filling of the present disclosure can include any of the aforementioned blends and granulates that were described with reference to tableting, but they are not subjected to a final tableting step.

The crystalline polymorphs of Elacestrant dihydrochloride of the present disclosure can be used as medicaments, in embodiments in the treatment of breast cancer.

Having thus described the disclosure with reference to particular preferred embodiments and illustrative examples, those in the art can appreciate modifications to the disclosure as described and illustrated that do not depart from the spirit and scope of the disclosure as disclosed in the specification. The Examples are set forth to aid in understanding the disclosure but are not intended to, and should not be construed to limit its scope in any way.

Further aspects and embodiments of the present disclosure are set out in the numbered clauses below:

### Embodiment section

Aspects, advantageous features and preferred embodiments of the present invention are summarized in the following items:
1. A crystalline Elacestrant dihydrochloride which is characterized by data selected from one or more of the following:
   (i) an X-ray powder diffraction pattern substantially as depicted in Figure 1;
   (ii) an X-ray powder diffraction pattern having peaks at 5.7, 8.0, 12.2, 12.6, and 22.6 degrees 2-theta ± 0.2 degrees 2-theta
   (iii) a DSC curve substantially as depicted in Figure 2
   (iv) a TGA curve substantially as depicted in Figure 3
   (v) a DSV curve substantially as depicted in Figure 4
   or any combination of (i)-(v)
2. The crystalline Elacestrant dihydrochloride according to item 1, which is a hydrate.
3. The crystalline Elacestrant hydrochloride according to any one of items 1 or 2 dissolves in water at a concentration of about 34.5 mg/ml at 24 °C.
4. The crystalline Elacestrant hydrochloride according to any one of items 1 or 3 in substantially pure form.
5. A crystalline Elacestrant dihydrochloride which is characterized by data selected from one or more of the following:
   (i) an X-ray powder diffraction pattern substantially as depicted in Figure 5;
   (ii) an X-ray powder diffraction pattern having peaks at 8.7, 10.1, 13.0, 16.3, and 21.8 degrees 2-theta ± 0.2 degrees 2-theta
   (iii) a DSC curve substantially as depicted in Figure 6
   (iv) a TGA curve substantially as depicted in Figure 7
   (v) a DSV curve substantially as depicted in Figure 8
   or any combination of (i)-(v).
6. The crystalline Elacestrant dihydrochloride according to item 5, which is a hydrate.
7. The crystalline Elacestrant dihydrochloride according to items 5 and 6, which is a dihydrate.
8. The crystalline Elacestrant hydrochloride according to any one of items 5 to 7 which dissolves in water at a concentration of about 51.9 mg/mL at 24 °C.
9. The crystalline Elacestrant hydrochloride according to any one of items 5 to 9 in substantially pure form.
10. A pharmaceutical composition comprising a crystalline product according to any of items of 1 to 9.
11. A pharmaceutical composition comprising a crystalline product according to any of items of 5 to 9 and amorphous Elacestrant dihydrochloride.
12. Use of a crystalline product according to any of items 1 to 9 for the preparation of a pharmaceutical composition and/or pharmaceutical formulation.
13. A pharmaceutical formulation comprising a crystalline product according to any of items 1 to 9, or a pharmaceutical composition of items 10 or 11, with at least one pharmaceutically acceptable excipient.
14. A crystalline product according to any of items 1 to 9, a pharmaceutical composition according to items 10 or 11, or a pharmaceutical formulation according to Claim 24, for use as a medicament.
15. A crystalline product according to any of items 1 to 9, for use according to item 14, wherein the medicament is for the treatment of breast cancer
17. A method of treating of breast cancer comprising administering a therapeutically effective amount of a crystalline product according to any of items 1 to 9, a pharmaceutical composition according to item 10 or 11, or a pharmaceutical formulation according to item 14, to a subject in need of treatment.
18. A process of preparing crystalline Elacestrant hydrochloride according to any one of items 1 to 4 comprising the step of
   (i) dispersing amorphous Elacestrant free base in a solvent
   (ii) adding hydrochloride acid and stirring until crystallization has completed
   (iii) isolating the crystals obtained in (ii)
   (iv) drying the isolated crystals obtained in (iii)
   (v) optionally milling the crystals obtained in (iv)
   (vi) optionally sieving the crystals of obtained in (iv, v).
19. The process wherein the solvent is selected from alcohols, preferentially the solvent is selected from C2-C6 aliphatic alcohols, most preferentially the solvent is 2-propanol or 1-butanol.
20. The process wherein aqueous hydrochloric acid added, preferentially about 37% aqueous hydrochloric is added.
20. The process wherein the solution is stirred for a period within a range from about 12 hours to about 24 hours, preferentially within a range from about 16 hours to about 20 hours, most preferentially about 18 hours.
21. The process wherein the crystals of Elacestrant dihydrochloride according to items 1 to 4 obtained in (ii) are isolated by conventional methods such as filtering or centrifugation.
22. The process wherein the isolated crystals of Elacestrant dihydrochloride according to items 1 to 4 obtained in (iii) are optionally washed with a solvent selected from the groups of aliphatic alcohols, ketones, and ethers, preferentially the solvent is selected from C2-C6 aliphatic alcohols, most preferentially the solvent is 2-propanol or 1-butanol.
23. The process wherein isolated crystals of Elacestrant dihydrochloride according to items 1 to 4 obtained in (iii, iv) are dried for a period within a range from about 12 hours to about 24 hours, preferentially within a range from about 16 hours to about 20 hours, most preferentially about 18 hours.
24. The process wherein isolated crystals of Elacestrant dihydrochloride according to items 1 to 4 obtained in (iii, iv) are dried at a temperature within a range from about 10 °C to about 30 °C, preferentially within a range from about 15 °C to about 25 °C, most preferentially at RT.
25. The process wherein the particle size distribution of the crystals of Elacestrant dihydrochloride according to items 1 to 4 obtained in (v, vi) is optionally controlled by a milling and/or sieving process.
26. A process of preparing crystalline Elacestrant hydrochloride according to any one of items 5 to 9 comprising the step of
   (i) storing amorphous Elacestrant dihydrochloride at a humidity higher than 60% and at a temperature below 40 °C
   (ii) optionally checking crystallinity
   (iii) optionally milling the crystals obtained in (i);
   (iv) optionally sieving the crystals obtained in (i, ii).
27. The process wherein the amorphous Elacestrant dihydrochloride is stored at a humidity in a range of from about 70% to 85%, preferable from about 70% to 85%, at a temperature in a range of from about 15 °C to 35 °C, preferably, in a range of from about 20 °C to 30 °C. Most preferable the amorphous Elacestrant hydrochloride is stored at a humidity in a range of from about 75% to 80% at RT.
28. The process wherein the amorphous Elacestrant dihydrochloride is stored for a period in a range of from about 3 to 10 days, preferable from about 5 to 8 days, most preferable from about 6 to 7 days.
29. The process wherein the crystallinity is optionally checked by PXRD.
30. The process wherein the particle size distribution of the crystals of Elacestrant dihydrochloride according to items 5 to 10 obtained in (i) is optionally controlled by a milling and/or sieving process.

The following non-limiting examples are illustrative of the disclosure and are not to be construed to be limiting to the scope of the claims.

### EXAMPLES

### Preparatory Examples

Elacestrant free base was prepared according WO 2004/058682. Amorphous Elacestrant dihydrochloride was prepared according to WO 2016/176665.

### Example 1

Elacestrant free base (50 mg) was suspended at room temperature in of 2-propanol (0.5mL). After addition of hydrochloride acid (37%; 0.1 mL) at RT a solution was obtained. The solution was stored at RT overnight and the resulting solid product was isolated by centrifugation. The wet product was dried atmosphere at room temperature under atmospheric pressure to yield Elacestrant dihydrochloride form A.

### Example 2

Elacestrant free base (50 mg) was suspended at room temperature in of 1-butanol (0.5mL). After addition of hydrochloride acid (37%; 0.1 mL) at RT a solution was obtained. The solution was stored at RT overnight and the resulting solid product was isolated by centrifugation. The wet product was dried atmosphere at room temperature under atmospheric pressure to yield Elacestrant dihydrochloride form A.

### Example 3

Amorphous Elacestrant dihydrochloride were stored at 75 - 80% relative humidity at room temperature in a ProUmid powder conditioner for 6 days to yield Elacestrant dihydrochloride form B.

### Analytical examples

### Example 1. Powder X-ray diffraction

Powder X-ray diffraction was performed with a PANalytical X'Pert PRO diffractometer equipped with a theta/theta coupled goniometer in transmission geometry, Cu-Kalpha1,2 radiation (wavelength 0.15419 nm) with a focusing mirror and a solid state PIXcel detector. Diffractograms were recorded at a tube voltage of 45 kV and a tube current of 40 mA, applying a step size of 0.013° 2-theta with 40s per step (255 channels) in the angular range of 2° to 40° 2-Theta at ambient conditions. A typical precision of the 2-Theta values is in the range of ± 0.2° 2-Theta, preferably of ± 0.1° 2-Theta.

Form A: significant peaks were observed at the following positions [± 0.2 °2Th.] (list 1): 5.7; 8.0; 10.1; 11.3; 12.2; 12.6; 13.1; 14.5; 14.9; 16.0; 18.0; 18.5; 18.8; 19.3; 20.0; 20.7; 21.0; 21.5; 22.1; 22.6; 24.1; 24.7, 25.0; 25.8; 26.2; 26.5; 27.4; 27.9; 28.3; 28.8; 29.2; 30.0; 30.3; 30.9; 31.8; 32.1; 32.6; 34.2; 34.9.

Form B: significant peaks were observed at the following positions [± 0.2 °2Th.] (list 2): 6.0; 8.7; 10.1; 10.5; 11.0; 12.1; 13.0; 13.4; 15.8; 16.0; 16.3; 16.9; 17.5; 18.0; 18.5; 18.9; 20.0; 20.9; 21.8; 23.1; 23.4; 24.3; 24.5; 24.9; 25.4; 25.7; 26.5; 27.3; 28.7; 29.2; 30.1; 30.7.

### Example 2. Differential Scanning Calorimetry (DSC)

DSC was performed on a Mettler Polymer DSC R instrument. The sample was heated in a 40 microliter aluminum pan with a pierced aluminum lid from 25 to 200 °C at a rate of 10 K/min. Nitrogen (purge rate 50 mL/min) was used as purge gas. Form A: the DSC curve shows more endothermic peaks with an onset temperature of about 68 °C and 177 °C a peak maximum temperature of about 93,5 °C and 190 °C and is displayed in Figure 2.

Form B: the DSC curve shows more endothermic peaks with an onset temperature of about 68 °C, 152 °C and 213 °C a peak maximum temperature of about 95 °C, 164 °C and 225 °C. It shows also a exothermic peak with an onset of about 167 °C and a peak minimum temperature of about 170 °C and is displayed in Figure 6.

### Example 3. Thermogravimetric Differential Thermal Analysis (TG-DTA)

Thermogravimetric analyses were carried out on crystalline Elacestrant hydrochloride from example 1 on a Mettler Toledo TGA/DSC1 STARe. The calibration standards were indium and tin. Samples were placed in an aluminum sample pan, inserted into the TG furnace and accurately weighed. The heat flow signal was stabilized for one minute at 30°C, prior to heating to 270°C in a stream of nitrogen at a rate of 10°C/minute.

Form A: the DSV curve recorded is displayed in Figure 4.

Form B: the DSV curve recorded is displayed in Figure 8.

### Example 4. Dynamic Vapor Sorption (DVS):

Moisture sorption isotherms were recorded with an SPSx-1µ moisture sorption analyzer (ProUmid, Ulm). Measurement cycles were started at ambient relative humidity. Relative humidity was then decreased to 5% RH in 5% steps, followed by a further decrease to 3% RH and to approximately 1% RH. Afterwards RH was increased from 1% to 90% RH in a sorption cycle and decreased to 1 % in a desorption cycle in 5% steps.

Form A: the TGA curve shows a mass loss of 9,4% until 110°C and is displayed in Figure 3.

Form B: the TGA curve shows a mass loss of 5.8% until 110°C and is displayed in Figure 7.

### Example 4. Dissolution testing

Aqueous solubility was determined dissolution experiments of Elacestrant dihydrochloride of the present invention in water at 24°C.

Reference experiment: 9.21mg of anhydrous Elacestrant dihydrochloride Form 1 disclosed in WO 2016/176665 did not completely dissolve in 1000µl water at 24°C indicating that the solubility is less than 9,2mg/ml.

Form A: 12,09mg dissolved in 350µl water at 24°C and resulted a clear solution containing 34,5mg/ml of Elacestrant dihydrochloride.

Form B: 10.37mg dissolved in 200µl water at 24°C and resulted a clear solution containing 51.9mg/ml of Elacestrant dihydrochloride. However, the supersaturated solution was found to be instable as precipitation of Elacestrant dihydrochloride was observed shortly after its formation.

Reference experiment: 9.21mg of anhydrous Elacestrant dihydrochloride Form 1 disclosed in WO 2016/176665 did not completely dissolve in 1000µl water at 24°C indicating that the solubility is less than 9,2mg/ml.

### Result:

Crystalline polymorphs of Elacestrant dihydrochloride of the present invention show a significant improved dissolution behavior in comparison with anhydrous Elacestrant dihydrochloride Form 1 used in ORSERDU^{™} tablets.

### Example 5. Stability testing

Preliminary stability testing for crystalline polymorphs of Elacestrant dihydrochloride of the present invention after 1, 2 and 4 weeks storage at 40°C/75%rH suggest no substantial changes in the XRPD pattern. Further studies are ongoing.

## Claims

1. Crystalline Elacestrant dihydrochloride which is **characterized by** data selected from one or more of the following:
(i) an X-ray powder diffraction pattern substantially as depicted in Figure 1;
(ii) an X-ray powder diffraction pattern having peaks at 5.7, 8.0, 12.2, 12.6, and 22.6 degrees 2-theta ± 0.2 degrees 2-theta
(iii) a DSC curve substantially as depicted in Figure 2
(iv) a TGA curve substantially as depicted in Figure 3
(v) a DSV curve substantially as depicted in Figure 4
or any combination of (i)-(v)

2. Crystalline Elacestrant dihydrochloride which is **characterized by** data selected from one or more of the following:
(i) an X-ray powder diffraction pattern substantially as depicted in Figure 5;
(ii) an X-ray powder diffraction pattern having peaks at 8.7, 10.1, 13.0, 16.3, and 21.8 degrees 2-theta ± 0.2 degrees 2-theta
(iii) a DSC curve substantially as depicted in Figure 6
(iv) a TGA curve substantially as depicted in Figure 7
(v) a DSV curve substantially as depicted in Figure 8
or any combination of (i)-(v).

3. The crystalline Elacestrant hydrochloride according to claim 1, 2, which is a hydrate.

4. The crystalline Elacestrant hydrochloride according to any one of claims 1 or 2 in substantially pure form.

5. A pharmaceutical composition comprising a crystalline product according to any of Claims of 1 to 4.

6. Use of a crystalline product according to any of Claims 1 to 4 for the preparation of a pharmaceutical composition and/or pharmaceutical formulation.

7. A pharmaceutical formulation comprising a crystalline product according to any of Claims 1 to 4, or a pharmaceutical composition of Claim 6, with at least one pharmaceutically acceptable excipient.

8. A crystalline product according to any of Claims 1 to 4, a pharmaceutical composition according to Claim 6, or a pharmaceutical formulation according to Claim 7, for use as a medicament.

9. A crystalline product according to any of Claims 1 to 4, for use according to Claim 8, wherein the medicament is for the treatment of breast cancer.

10. A method of treating of breast cancer comprising administering a therapeutically effective amount of a crystalline product according to any of Claims 1 to 4, a pharmaceutical composition according to Claim 6, or a pharmaceutical formulation according to Claim 7, to a subject in need of treatment.

11. A process of preparing crystalline Elacestrant hydrochloride according to Claim 1 comprising the step of
(i) dispersing amorphous Elacestrant free base in a solvent
(ii) adding hydrochloride acid and stirring until crystallization has completed
(iii) isolating the crystals obtained in (ii)
(iv) drying the isolated crystals obtained in (iii)
(v) optionally milling the crystals obtained in (iv)
(vi) optionally sieving the crystals of obtained in (iv, v).

12. A process of preparing crystalline Elacestrant hydrochloride according to Claim 2 comprising the step of
(i) storing amorphous Elacestrant dihydrochloride at a humidity higher than 60% and at a temperature below 40 °C
(ii) optionally checking crystallinity
(iii) optionally milling the crystals obtained in (i);
(iv) optionally sieving the crystals obtained in (i, ii).
